# EUROPEAN PATENT SPECIFICATION

(11) **EP 1 174 158 B1**
(45) Date of publication and mention of the grant of the patent: **27.10.2004**
(21) Application number: 01116001.7
(22) Date of filing: 30.06.2001
(51) Int. Cl.: A61M 5/50, A61M 5/32

(54) **Safety syringe**
Sicherheitsspritze
Seringue de sécurité

(30) Priority: 18.07.2000 ZA 200003593
(43) Date of publication of application: 23.01.2002
(73) Proprietor: VENTRADEX AG, 5056 Attelwil (CH)
(72) Inventor: Du Plessis, Johannes S., Lichtenburg, North West Province (ZA)
(74) Representative: Morva, Tibor

(56) References cited:
- EP-A- 0 438 368
- AT-B- 395 679
- FR-A- 2 741 268
- US-A- 5 788 677

## Description

The present invention relates to a syringe, thus of the type comprising a chamber with tubular cylindrical configuration. The plunger means within the said tubular cylindrical configuration being able of moving along the axial axis of the tubular cylindrical configuration and a needle holder configuration fixed externally or as an integral part to the discharge end of the tubular cylindrical configuration of the syringe opposite that of the plunger means handle.

The said needle holder configuration connects to the discharge end of the syringe by means of a one piece receptacle module, allowing said connection to be done from externally means, or as an integral part of the syringe tubular configuration, and whereby it is made possible to cover and protect the protrusive needle portion after medicament discharge by means of a protruding shield extending internally from the tubular cylindrical configuration of the syringe chamber front end, thus the discharge end, actuated by means of the force exerted on the rear end of the plunger means handle directly after medicament discharge, in the same axial direction as for medicament discharge, to the most forward position of the plunger means on the discharge end of the plunger means handle to counter any accidental contact with the needle portion during further handling.

Other embodiments of such conventional safety syringes may have the following shortcomings, such as that,
1. the needle holder configuration or device has to be inserted from the non-discharge end thus the rear end of the syringe,
2. needles of different sizes cannot be accommodated on the syringe,
3. increased production costs and assembling difficulties due to plug type embedments into the discharge end of the syringe,
4. a considerable degree of complexity as if for special applications only,
5. un-official re-use possibility of syringe System after initial usage, thus causing extreme un-safe conditions, due to a slight re-activation force onto certain components of the syringe System so as to reconstruct the System as if for initial use.

By the FR-A1-2741268 is described a pre-filled syringe with a protected needle of variable length. This syringe incorporates a mobile guard to hold and guide the needle, along which it slides after manually releasing lugs engaging with holes in a holder to determine the length of the needle to be employed for an injection or to cover it completely at the end of the plunger's injection stroke. The guard, the holder and a needle cap form a sealed assembly which isolates the tip of the needle from external contamination during storage. This concept cannot make possible the intake of medicament via needle point and piston where the piston is a partition between the back of the needle and the medicament. The tube containing the medicament is not rigidly connected to the holder, only via the piston in the tube. This syringe is a risky apparatus. This syringe may be re-used since the mobile guard may be forcibly removed and a second or more pre-filled vials connected individually in turn to the rear end of this syringe. There is no additional destruction or disablement other than the guard to the main body comprising the syringe handle. The length of the needle depends on the useful length of the vial, thus the need for a guard. An air hole is provided in the tube, in the main structure of which the medicament is contained. Further more, if this vent hole is closed off prior to usage, then this syringe may be used indefinitely. This allows that the piston in the tube may not be fully pressed into the far end of the tube. This may thus not fully activate and locks the guard into its locked position.

The aim of this invention is to avoid the mentioned disavantages of the known conventional syringe.

However according to the present invention, a safety syringe is provided accommodating a configurative needle holder connected to, or as an integral part of the tubular cylindrical discharge end of the syringe also accommodating a receptor and sealing means to form the discharge end seal means. This includes a plunger means, slidably contained in the syringe incorporating a use once only, plunger means seal to prevent further use of sealing means principal on plunger means. The plunger means cylindrical section communicating directly after medicament discharge, with the receptor means thus de-activating the sealing means of the needle holder configurative means including the plunger means sealing means almost simultaneously during the further axial movement in the direction of the discharge end thus the needle portion, to a fixed distance traveled so to stop against the most forward stop means for the receptor means to such an extent that the receptor means is locked in the most forward position through which a shield is permanently provided to cover the entire needle portion.

The entire syringe mechanism is thus rendered useless in that the entire needle portion is shielded off against any accidental contact and the plunger means is thus also destructively changed so as not to re-seal within the tubular cylindrical configuration of the syringe thus leaving both the discharged end and the rear end of the syringe inoperable to neither allow the possibility of suction nor compression during any further Operation of the plunger means handle means. Based on the configuration explained, and during the plunger means advancement during the injection phase and when reaching the most forward Position to fully discharge the medicament, the cylindrical section of said plunger means, when continue pressing the plunger means handle towards the discharge end of the syringe, communicating directly with the non-discharge end of the receptor means cylindrical section, preferably to the same outer diameter and seal diameter as the plunger means, but slightly larger in cylindrical internal diameter than the outer diameter of the rear end of the configurative needle holder portion so as to allow without binding movement on the said needle holder portion, thus allowing receptor means to be pushed forward towards the discharge end of the syringe to such a position, as to lock in its most forward position thus providing a shield around the protrusive needle portion to render the said needle portion protected during the said further handling of the said syringe.

During the stage when the plunger means is pushed towards the discharge end of the syringe, just after complete medicament discharge, and when the cylindrical portion of the plunger means is moving against the cylindrical portion of the receptor means and thus moving axially and slidably over the rear end portion of the configurative needle holder and during the de-activation of the receptor means seal means and configurative means needle holder seal means, the seal means of the hollow cylindrical plunger means is likewise de-activated such that no seal means further exists to form a closed cylindrical chamber which form the basis of a syringe internal chamber principal into which a volume of liquid may be contained.

From this position the advancement of the plunger means handle may proceed to lock the receptor means in its most forward position.

At this stage the syringe is rendered useless to any further use and may thus be discarded.

The above mentioned characteristics will be further understood upon the applicable description, in which reference is made to attached drawings

Revealing possible variants of the invention, but these only been given simply as Examples and should thus not be understood as limited to these.

The present invention will thus be further described with said reference to the said drawings, in which:
Figure 1 is a sectional view showing all parts in construction of a syringe in accordance with the invention with the plunger means in its re-arranged layout after medicament discharge and with the needle protector in its most forward position.
Figure 2 is a sectional view showing all parts in construction of a syringe in accordance with the invention and in a pre-use layout.

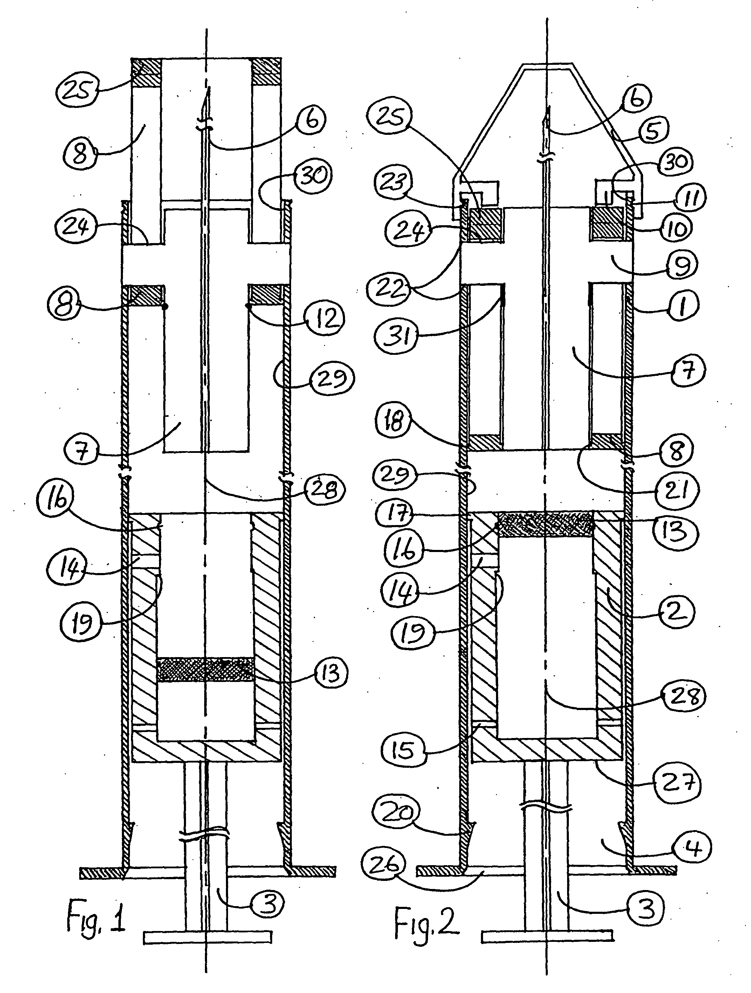

As shown in figures 1 and 2, the present invention comprises the syringe, having a cylindrical chamber 1; with which is introduced a plunger means 2, capable of moving axially in a hollow cylindrical portion 29, open on its rear end 4. Its front end forms and open cylindrical end 30, into which a cylindrical slidable seal means 8 is contained partially around a needle holder means 7 which forms the closure to the syringe front end 30. The needle holder means 7 and the cylindrical slidable seal means 8 is of an interconnected type attachably connected to the cylindrical chamber 1 by means of the assembled needle holder means 7 into the cylindrical slidable seal means 8 into the cylindrical chamber means 1 by means of the insertion of least one male protrusion 9 into at least one female receptacle area 22 to form the front end thus the discharge end of the syringe means. The needle 6 is protected prior to use, by means of a protective cap 5 remaining in place until time of use, whereby the protective cap 5 may be removed from cylindrical chamber 1 by means of a radial twist action on protective cap 5 to detachably release from said cylindrical chamber 1.

The syringe means 29 is for filling liquid medicament by means of a slidable action of the plunger means 2 towards the rear end 4 of cylindrical chamber 1. The plunger means 2, comprises of a plunger handle 3 on the rear end and a hollow cylindrical plunger head having a seal means 13 closing off the syringe discharge end of the plunger means 2, with specific configuration to contain seal means 13, restricted by means of a radial groove 16 to increase initial slidable friction. Seal means 13 is pushed to rear end 4 after complete medicament discharge when pushing plunger handle means 3 towards the syringe discharge end, and in the same cylindrical axes 28, by means of the rear end of needle holder 7, to a fixed position whereby seal means 13 moving to the rear end 4 passing over and beyond aperture 14 so as to open the plunger means 2 thus to disable the sealing effect of the plunger means 2 thus regarding the said plunger means 2 incapable for further sealing, suction or compression.

When sealing means 13 is pushed towards the rear end 4, the air escape routes 15 allows normal atmospheric pressure inside the rear end portion of plunger means 7 and the rear end face of seal means 13, and the plunger means 2 is thus rendered useless for further use, since seal means 13 cannot move towards the discharge end of the syringe by means of a natural slidable movement since seal means 13 is in this condition slightly larger in outer diameter than the internal diameter starting at diametrical step change 19.

During the actual movement of the plunger means 2 towards the discharge end of the syringe, plunger means 2 moves slidable over the rear end portion of the needle holder 7 at lesser outer diameter than the internal diameter of plunger means 2, causing the cylindrical slidable seal means 8 to be pushed forward in the direction of the syringe discharge to the most forward position where the cylindrical slidable means 8 is positively locked onto the fixed needle holder 7 front end portion at, at least one lock means 12. At this stage the cylindrical slidable means 8 has protruded to the most forward position to a fixed distance thus covering and protect the needle 6 in excess distance than the protrusive length of needle 6.

In this position the seal means 21 of cylindrical slidable seal means 8 on needle holder 7 and seal means 18 of cylindrical slidable seal means 8 on hollow cylindrical portion 29 is likewise rendered useless due to a lesser diameter interface between the said interfaces due to a slight cylindrical step change 31 on needle holder 7 at positive lock 12.

The entire syringe is thus rendered useless due to the destruction of the sealing interface between the seal means 13 and the internal diameter of plunger means 2 and the inadequate seal means between the cylindrical chamber 1, the cylindrical slidable seal means 8 and the needle holder 7. Plunger means 2 is stopped positively on the most rearward cylindrical face 27 against stop 20 provided integral in the construction of cylindrical chamber 1. The taper edge 26 is provided to allow easy access into the cylindrical chamber 1 for plunger means 2. A seal means 17 is provided on plunger means 2 outer diameters to allow for a seal means between the plunger means 2 and the hollow cylindrical portion 29. The cylindrical slidable seal means 8 front end 25 has a positive stop 24 on at least one male protrusion 9 in its most rearward configurative position prior to use.

Accidental activation of the syringe configuration prior to use, is countered by having cylindrical slidable seal means 8 front cylindrical stop section 25 stopped against at least one stop means 10 an integral part of the needle protective cap 5, held in position prior to use by means of at least one male protrusion 11, engaged into at least one female aperture 23, thus forming the front stop means for cylindrical slidable means 8.

## Claims

1. A safety syringe comprising a cylindrical chamber (1), a plunger means (2) reciprocatively held in the cylindrical chamber (1), a needle holder (7) and cylindrical slidable seal means (8) connected to the front end (30) of the cylindrical chamber (1), the said plunger means (2) with cylindrical configuration to contain internally in the plunger means(2) a seal means (13) which is slidably moved rearward after complete medicament discharge by means of the rear end portion of the needle holder (7) when plunger means (2) is pushed forward towards the discharge end of the syringe after medicament discharge whereby cylindrical slidable seal means (8) is pushed forward by the said plunger means (2) towards the discharge end of the syringe until the said cylindrical slidable seal means (8) is locked positively onto the needle holder (7) portion against accidental injuries at which stage the plunger means (2) is simultaneously rendered useless by means of the dislodged seal means (19) internally to the plunger means (2) by open configurative layout in the plunger means (2) thus having an open ended plunger means (2) without the possibility of re-use as a plunger means (2).

2. Syringe according to claim 1, **characterised in that** the sealing means on the cylindrical slidable seal means (8) comprises of at least one seal means (21) on the internal diameter and at least one seal means (18) on the outer diameter of the said cylindrical slidable seal means (8) onto the needle holder (7) and onto the cylindrical chamber (1) to form the discharge end seal means principal between the needle holder (7) portion and the cylindrical chamber (1) portion.

3. Syringe according to claims 1 and 2, **characterised in that** the plunger means (2) comprises of at least one seal means (17) on the outer diameter onto the cylindrical chamber (1) and at least one seal means (16) on the inner diameter of the plunger means (2) onto the internal seal means (13).

4. Syringe according to any of the preceding claims, **characterised in that** the said syringe is not limited to a single lenght needle portion (6) and holder (7).

5. Syringe according to any of the preceding claims, **characterised in that** the plunger means (2) is not limited to a hollow configuration plunger with at least one seal means (13), (16), (17).

6. Syringe according to any of the preceding claims, **characterised in that** the syringe means be adapted under the same pricipal as the said spirit of this invention and specification, also at least to allow for pre-filled option on any of the version as here-in described.

## Patentansprüche

1. Sicherheitsspritze, bestehend aus einer zylindrischen Kammer (1), einem in der zylindrischen Kammer (1) in beide Richtungen verschiebbar geführten Kolben (2), einem Kanülenhalter (7) und einer zylindrischen verschiebbaren Dichtung (8), die mit dem vorderen Ende (30) der zylindrischen Kammer (1) verbunden ist, wobei im Inneren des zylindrisch konfigurierten Kolbens (2) eine Dichtung (13) vorgesehen ist, die beim Hineindrücken des Kolbens (2) in Richtung des Ausgabeendes der Spritze, wobei die zylindrische verschiebbare Dichtung (8) durch den Kolben (2) nach vorne in Richtung des Ausgabeendes der Spritze gedrückt wird, bis sie zur Vermeidung von Unfallverletzungen am Kanülenhalter (7) formschlüssig einrastet, nach erfolgter Arzneimittelabgabe durch den hinteren Endabschnitt des Kanülenhalters (7) nach rückwärts verschoben wird, womit der Kolben (2) in Folge seiner offenen Konfiguration aufgrund der ins Innere des Kolbens (2) hineinverschobenen Dichtung (19) unbrauchbar wird, da ein Kolben (2) mit offenem Ende nicht mehr als Kolben (2) wiederverwendbar ist.

2. Spritze nach Anspruch 1, **dadurch gekennzeichnet, dass** die Dichtung auf der zylindrischen verschiebbaren Abdichtung (8) mindestens eine Dichtung (21) am Innenumfang und mindestens eine Dichtung (18) am Außenumfang der zylindrischen verschiebbaren Dichtung (8) umfasst, die am Kanülenhalter (7) bzw. an der zylindrischen Kammer (1) anliegen, um zwischen dem Kanülenhalter (7) und der zylindrischen Kammer (1) die Hauptdichtung am Ausgabeende zu bilden.

3. Spritze nach Ansprüchen 1 und 2, **dadurch gekennzeichnet, dass** der Kolben (2) am Außenumfang mindestens eine an der zylindrischen Kammer (1) anliegende Dichtung (17) und an seinem Innenumfang mindestens eine an der Innendichtung (13) anliegende Dichtung (16) umfasst.

4. Spritze nach einem der vorhergehenden Ansprüche, **dadurch gekennzeichnet, dass** sie nicht auf Kanülen (6) und Halter (7) einer einzigen Länge beschränkt ist.

5. Spritze nach einem der vorhergehenden Ansprüche, **dadurch gekennzeichnet, dass** der Kolben (2) nicht auf eine Hohlkolbenkonfiguration mit nur mindestens einer Dichtung (13), (16), (17) beschränkt ist.

6. Spritze nach einem der vorhergehenden Ansprüche, **dadurch gekennzeichnet, dass** die Spritze entsprechend dem Geist der vorliegenden Erfindung und der Beschreibung im Hinblick auf die gleichen Prinzipien adaptiert werden kann, um wenigstens für eine der hier beschriebenen Versionen auch die Alternative einer Fertigspritze zu ermöglichen.

## Revendications

1. Seringue de sécurité comprenant une chambre cylindrique (1), un moyen de plongeur (2) logé d'une façon réciproque dans la chambre cylindrique (1), un support d'aiguille (7) et un moyen de joint coulissant cylindrique (8) connecté à l'extrémité avant (30) de la chambre cylindrique (1), ledit moyen de plongeur (2) présentant une configuration cylindrique qui lui permet de contenir intérieurement dans le moyen de plongeur (2) un moyen de joint (13) qui est déplacé d'une façon coulissante vers l'arrière après l'expulsion complète du médicament au moyen de la partie d'extrémité arrière du support d'aiguille (7) lorsque le moyen de plongeur (2) est poussé vers l'avant en direction de l'extrémité de décharge de la seringue après l'expulsion du médicament, dans lequel le moyen de joint coulissant cylindrique (8) est poussé vers l'avant par ledit moyen de plongeur (2) en direction de l'extrémité de décharge de la seringue jusqu'à ce que ledit moyen de joint coulissant cylindrique (8) soit verrouillé positivement sur la partie de support d'aiguille (7) pour prévenir des blessures accidentelles, moment auquel le moyen de plongeur (2) est simultanément rendu inutilisable au moyen du moyen de joint délogé (19) intérieurement sur le moyen de plongeur (2) par la disposition de configuration ouverte dans le moyen de plongeur (2), présentant ainsi un moyen de plongeur à extrémité ouverte (2) sans possibilité de le réutiliser comme moyen de plongeur (2).

2. Seringue suivant la revendication 1, **caractérisée en ce que** le moyen d'étanchéité sur le moyen de joint coulissant cylindrique (8) comprend au moins un moyen de joint (21) sur le diamètre intérieur, et au moins un moyen de joint (18) sur le diamètre extérieur dudit moyen de joint coulissant cylindrique (8) sur le support d'aiguille (7) et sur la chambre cylindrique (1) afin de former le moyen de joint principal de l'extrémité de décharge entre la partie de support d'aiguille (7) et la partie de chambre cylindrique (1).

3. Seringue suivant les revendications 1 et 2, **caractérisée en ce que** le moyen de plongeur (2) comprend au moins un moyen de joint (17) sur le diamètre extérieur sur la chambre cylindrique (1), et au moins un moyen de joint (16) sur le diamètre intérieur du moyen de plongeur (2) sur le moyen de joint intérieur (13).

4. Seringue suivant l'une quelconque des revendications précédentes, **caractérisée en ce que** ladite seringue n'est pas limitée à une seule longueur de partie d'aiguille (6) et de support d'aiguille (7).

5. Seringue suivant l'une quelconque des revendications précédentes, **caractérisée en ce que** le moyen de plongeur (2) n'est pas limité à une configuration creuse du plongeur avec au moins un moyen de joint (13), (16), (17).

6. Seringue suivant l'une quelconque des revendications précédentes, **caractérisée en ce que** le moyen de seringue est en outre adapté, sous le même principe et conformément audit esprit de cette invention et de cette description, de manière à au moins permettre l'application d'une option de pré-remplissage sur l'une quelconque des versions décrites ici.
